# EUROPEAN PATENT SPECIFICATION

(11) **EP 2 361 118 B1**
(45) Date of publication and mention of the grant of the patent: **30.08.2017**
(21) Application number: 09764253.2
(22) Date of filing: 02.12.2009
(51) Int. Cl.: A61Q 5/00, A61Q 5/02, A61Q 5/12, A61K 8/85, A61K 8/86, A61K 8/88

(54) **HAIR CARE COMPOSITION COMPRISING A DENDRITIC MACROMOLECULE**
HAARPFLEGEZUSAMMENSETZUNG MIT EINEM DENDRITISCHEN MAKROMOLEKÜL
COMPOSITION POUR LE SOIN DES CHEVEUX COMPRENANT UNE MACROMOLÉCULE DENDRITIQUE

(30) Priority: 22.12.2008 EP 08172498
(43) Date of publication of application: 31.08.2011
(73) Proprietor: Unilever PLC, Blackfriars London Greater London EC4Y 0DY (GB); Unilever N.V., 3013 AL Rotterdam (NL)
(72) Inventor: DERICI, Leo, Wirral Merseyside CH63 3JW (GB); HARCUP, Jason, Peter, Connecticut 06611 (US); HILL, Stuart, Paul, Wirral Merseyside CH63 3JW (GB); KHOSHDEL, Ezat, Wirral Merseyside CH63 3JW (GB)
(74) Representative: Chisem, Janet
(86) International application number: PCT/EP2009/066255
(87) International publication number: WO 2010/072527

(56) References cited:
- WO-A-01/17485
- WO-A-2006/018063
- WO-A-2006/018064
- WO-A-2006/018641
- US-A1- 2007 202 071

## Description

The present invention relates to a hair care composition comprising non-linear polymers.

US 2007/202071 discloses compositions comprising dendritic molecules as emulsifying agents. However, by their physical nature such dendritic molecules are not capable of delivering the benefits of the invention.

In addition, whereas dendritic macromolecules decreasing the volume of hair have been disclosed before in hair care compositions, the invention shows that compositions in which the hydrophobically modified dendritic macromolecule is emulsified prior to incorporation into the composition have even lower volume compared to those compositions with dendritic macromolecules that are not emulsified.

Accordingly, the present invention relates to a hair care composition according to claim 1.

Dendritic macromolecules are macromolecules with densely branched structures having a large number of end groups. A dendritic polymer includes several layers or generations of repeating units which all contain one or more branch points. Dendritic polymers, including dendrimers and hyperbranched polymers, are prepared by condensation reactions of monomeric units having at least two different types of reactive groups. Dendrimers are highly symmetric, whereas macromolecules designated as hyperbranched may to a certain degree hold an asymmetry, yet maintaining the highly branched treelike structure.

Dendritic macromolecules normally consist of an initiator or nucleus having one or more reactive sites and a number of branching layers and optionally a layer of chain terminating molecules. Continued replication of branching layers normally yields increased branch multiplicity and, where applicable or desired, increased number of terminal groups. The layers are usually called generations and the branches dendrons.

The dendritic macromolecule is hydrophobically modified and is a hydroxyl-functionalised dendritic macromolecule and one which comprises polyester units.

Suitable materials are described in SE 468 771, which discloses a macromolecule which is composed of an initiator, having at least one hydroxyl group, to which initiator is added at least one branching generation comprising at least one chain extender, having at least one carboxyl group and at least two hydroxyl groups.

Further suitable materials are described in SE 503 342, in which the macromolecule is substantially composed of a nucleus, having at least one epoxide group, to which nucleus is added at least one branching generation comprising at least one chain extender, having at least three reactive functions of which at least one is a carboxyl or epoxide group and at least one is a hydroxyl group.

These materials are also referred to as polyhydric polyester alcohols or hyperbranched polyols. Preferably these materials have at least eight, more preferably at least sixteen, most preferably at least thirty-two terminal hydroxyl groupings per macromolecule. Their molecular weight is preferably at least 800, more preferably at least 1600, most preferably at least 2500 g/mole.

These materials are available commercially from Perstorp AB, SE-284 80 Perstorp, Sweden under the trademark of BOLTORN Examples of such materials are BOLTORN H10, BOLTORN H20, BOLTORN H30 and BOLTORN H40, of which BOLTORN H30 and BOLTORN H40 are preferred.

It is preferred if the generation number of the polymer is 2 or greater. The maximum generation number is preferably 9 or less, more preferably 7 or less. The hydrophobically functionalised dendritic macromolecules are built up from polyester units. Suitable macromolecules of this type are disclosed in US 5 418 301 and can be sold under the tradename Perstop.

The level of hydrophobically functionalised dendritic macromolecule is preferably from 0.0001 to 30 wt% of the total composition, more preferably the level is from 0.05 to 8 wt%, most preferably from 0.1 to 5 wt%.

Preferably the number average molecular weight of the polymers are from 500 to 50,000, more preferably the number average molecular weight to from 500 to 10,000; most preferably the number average molecular weight is from 750 to 5,000.

### Hydrophobic modification

Preferred hydrophobic groups are carbon based. C₄-C₂₄ alkyl or alkenyl groups are preferred hydrophobic groups, more preferred are C₆-C₂₂ alkyl or alkenyl groups, especially preferred are C₈-C₁₆ alkyl or alkenyl groups, most preferred are dendritic macromolecule having C₁₀-C₁₄ alkyl or alkenyl groups. The hydrophobic groups may include linear and branched hydrophobes as well as arylalkyl groups, however it is preferred if the alkyl hydrophobic groups are linear. The hydrophobic groups may be unsaturated groups but are preferably saturated. The hydrophobic groups are sometimes linked to the dendritic macromolecule through linking groups, suitable linking groups include ester or amide groups.

In some instances it is preferred if the dendritic macromolecule is fully or partially hydrophobically functionalised at the periphery and/or the terminal groups of the dendritic macromolecule. (In the context of the present invention the term periphery means the outer layer or edge of the dendritic macromolecule.)

Where the dendritic macromolecule is hydrophobically functionalised at the periphery preferably 5 to 95% of the terminal groups are hydrophobically functionalised, more preferably from 10 to 85%, most preferably from 20 to 60%.

In a further embodiment the number of hydrophobic groups can be expressed as a percentage of the potential sites on the dendritic macromolecule available for hydrophobic modification both on the periphery of the molecule and internally within the molecule. Preferably 10 to 90 % of these available sites are hydrophobically modified, more preferably 20 to 70% are hydrophobically modified.

Hydrophobic modification is a simple process of reacting with the relevant fatty acid, fatty ester or fatty anhydride.

Examples of hydrophobically modified dendritic polymers include those commercially available as Boltorn H2004 and Boltorn W3000.

Preferably the composition is a shampoo composition and comprises materials commonly found in shampoo compositions such as silicones; cleansing surfactants such as anionic surfactants, amphoteric surfactants, nonionic surfactants and mixtures thereof.

Preferably, the composition according to the invention is a conditioning composition. Conditioning compositions according to the invention will comprise conditioning surfactants and actives such as silicones, fatty materials selected from alcohols, acids, amides and mixtures thereof.

Preferably, the composition according to the invention is a rinse-off composition.

### Preparation of the dendritic macromolecule emulsion for incorporation into a formulation as described in Example 1

**Table 1 -Example 1**

| Ingredient | Active % wt. |
|---|---|
| Boltorn H2004 | 20.00 |
| Cetyl trimethylammonium chloride (CTAC) | 3.00 |
| Pluracare F77 | 3.00 |
| Water | To 100.00 |

Water and CTAC were added to a beaker and heated to 60 °c until dissolved. Pluracare F77 was added while maintaining temperature at 60 °c, stirring with a spatula until dissolved. The mixture was allowed to cool and then transferred to a Silverson high shear rotor/stator laboratory mixer on speed 1 to which Boltorn H2004 was slowly added. The speed was increased to 6 and mixed for a further 20 minutes.

The following examples are shampoo compositions according to the invention to which Boltorn H2004 and emulsified Boltorn H2004 were added.

**Table 2 - Shampoo Compositions**

| Ingredient | Active % wt. | | | | | | | |
|---|---|---|---|---|---|---|---|---|
| | **A** | | | | **B** | | | |
| **Examples** | **2** | **3** | **4** | **5** | **2** | **3** | **4** | **5** |
| Sodium laureth (1EO) sulphate (SLES 1EO) | 12.00 | 12.00 | 12.00 | 12.00 | 12.00 | 12.00 | 12.00 | 12.00 |
| Coco amidopropyl betaine (CAPB) | 1.60 | 1.60 | 1.60 | 1.60 | 1.60 | 1.60 | 1.60 | 1.60 |
| Guar hydroxypropyl trimonium chloride | 0.20 | 0.20 | 0.20 | 0.20 | 0.20 | 0.20 | 0.20 | 0.20 |
| Silicone emulsion | 2.00 | 2.00 | 2.00 | 2.00 | 2.00 | 2.00 | 2.00 | 2.00 |
| Boltorn H2004 | 0.10 | 0.50 | 1.0 | 2.0 | - | - | - | - |
| Emulsified Boltorn H2004 **(Example 1)** | - | - | - | - | 0.10 | 0.50 | 1.0 | 2.0 |
| Sodium chloride | q.s. | q.s. | q.s. | q.s. | q.s. | q.s. | q.s. | q.s. |
| Water and minors | To 100 | To 100 | To 100 | To 100 | To 100 | To 100 | To 100 | To 100 |

The following examples are conditioner compositions according to the invention to which Boltorn H2004 and emulsified Boltorn H2004 were added.

**Table 3 - Conditioner Compositions**

| Ingredient | Active % wt. | | | |
|---|---|---|---|---|
| | **A** | | **B** | |
| **Examples** | **6** | **7** | **6** | **7** |
| Lactic acid | 0.38 | 0.38 | 0.38 | 0.38 |
| Stearamidopropyl dimethyl amine | 1.25 | 1.25 | 1.25 | 1.25 |
| Behentrimonium chloride | 0.87 | 0.87 | 0.87 | 0.87 |
| Cetearyl alcohol (30/70 C16/18) | 5.00 | 5.00 | 5.00 | 5.00 |
| Silicone emulsion | 2.50 | 2.50 | 2.50 | 2.50 |
| Boltorn H2004 | 2.00 | 4.00 | - | - |
| Emulsified Boltorn H2004 **(Example 1)** | - | - | 2.00 | 4.00 |
| Water and minors | To 100 | To 100 | To 100 | To 100 |

**Table 4 - Silhouette volume results for shampoo compositions with Boltorn H2004 and emulsified Boltorn H2004**

| Example | Silhouette volume / mm² | |
|---|---|---|
| | A | B |
| 2 | 7520 | 6114 |
| 3 | 7742 | 6397 |
| 4 | 7763 | 6490 |
| 5 | 5461 | 3477 |

| | | |
|---|---|---|
| Example 2 - 0.10% Boltorn H2004 Example 3 - 0.50% Boltorn H2004 Example 4 - 1.00% Boltorn H2004 Example 5 - 2.00% Boltorn H2004 A Boltorn H2004 B Emulsified Boltorn H2004 | | |

The table shows that shampoo compositions with emulsified Boltorn H2004 provide a lower volume than those compositions with Boltorn H2004. The experiment was carried out on uncombed hair.

**Table 5 - Silhouette volume results for conditioner compositions with Boltorn H2004 and emulsified Boltorn H2004**

| Example | Silhouette volume / mm² | |
|---|---|---|
| | A | B |
| 6 | 5049 | 3984 |
| 7 | 4115 | 3651 |

| | | |
|---|---|---|
| Example 6 - 2.00% Boltorn H2004 Example 7 - 4.00% Boltorn H2004 A Boltorn H2004 B Emulsified Boltorn H2004 | | |

The table shows that conditioner compositions with emulsified Boltorn H2004 provide a lower volume than those compositions with Boltorn H2004. The experiment was carried out on uncombed hair.

## Claims

1. Hair care composition comprising an emulsified hydrophobically functionalised dendritic macromolecule which is a polyhydric polyester alcohol, which is emulsified prior to incorporation into the composition, wherein the dendritic macromolecule is hydroxyl-modified.

2. Composition according to claim 1 in which the hydrophobically functionalised groups of the dendritic macromolecule are situated at the terminals of the macromolecule.

3. Composition according to any preceding claim in which the level of hydrophobically functionalised dendritic macromolecule is from 0.0001 to 30 wt% of the total composition.

4. Composition according to any preceding claim which is a shampoo composition.

5. Composition according to any preceding claim which is a conditioning composition.

6. Composition according to any preceding claim which is a rinse-off composition.

## Patentansprüche

1. Haarpflegezusammensetzung, umfassend ein emulgiertes, hydrophob funktionalisiertes dendritisches Makromolekül, welches ein mehrwertiger Polyesteralkohol ist, der vor der Einverleibung in die Zusammensetzung emulgiert wird, wobei das dendritische Makromolekül Hydroxyl-modifiziert ist.

2. Zusammensetzung nach Anspruch 1, in welcher sich die hydrophob funktionalisierten Gruppen des dendritischen Makromoleküls an den Enden des Makromoleküls befinden.

3. Zusammensetzung nach irgendeinem vorhergehenden Anspruch, in welcher die Konzentration des hydrophob funktionalisierten dendritischen Makromoleküls 0,0001 bis 30 Gew.-% der Gesamtzusammensetzung beträgt.

4. Zusammensetzung nach irgendeinem vorhergehenden Anspruch, welche eine Shampoo-Zusammensetzung darstellt.

5. Zusammensetzung nach irgendeinem vorhergehenden Anspruch, welche eine Konditionierzusammensetzung darstellt.

6. Zusammensetzung nach irgendeinem vorhergehenden Anspruch, welche eine Abspülzusammensetzung darstellt.

## Revendications

1. Composition de soins des cheveux comprenant une macromolécule dendritique hydrophobiquement fonctionnalisée qui est un polyester alcool polyvalent, qui est émulsionné avant l'incorporation dans la composition, dans laquelle la macromolécule dendritique est hydroxyle-modifiée.

2. Composition selon la revendication 1, dans laquelle les groupes hydrophobiquement fonctionnalisés de la macromolécule dendritique sont situés aux terminaisons de la macromolécule.

3. Composition selon l'une quelconque des revendications précédentes, dans laquelle la teneur de macromolécule dendritique hydrophobiquement fonctionnalisée est de 0,0001 à 30 % en masse de la composition totale.

4. Composition selon l'une quelconque des revendications précédentes qui est une composition de shampoing.

5. Composition selon l'une quelconque des revendications précédentes qui est une composition d'après-shampoing.

6. Composition selon l'une quelconque des revendications précédentes qui est une composition sans rinçage.
